# EUROPÄISCHE PATENTSCHRIFT

(11) **EP 2 059 296 B1**
(45) Veröffentlichungstag und Bekanntmachung des Hinweises auf die Patenterteilung: **25.03.2015**
(21) Anmeldenummer: 07856410.1
(22) Anmeldetag: 06.12.2007
(51) Int. Cl.: A61N 1/05, A61N 1/39

(54) **VORRICHTUNG ZUR DEFIBRILLATION DES HERZENS**
DEVICE FOR THE DEFIBRILLATION OF THE HEART
DISPOSITIF DE DÉFIBRILLATION DU COEUR

(30) Priorität: 28.02.2007 DE 102007009716
(43) Veröffentlichungstag der Anmeldung: 20.05.2009
(73) Patentinhaber: Osypka, Peter, 79618 Rheinfelden-Herten (DE)
(72) Erfinder: Osypka, Peter, 79618 Rheinfelden-Herten (DE)
(74) Vertreter: Kunst, Manuel Nikolaus Johannes
(86) Internationale Anmeldenummer: PCT/EP2007/010605
(87) Internationale Veröffentlichungsnummer: WO 2008/104209

(56) Entgegenhaltungen:
- EP-A- 0 211 166
- EP-A- 0 590 431
- EP-A- 0 602 356
- WO-A-2004/091715
- US-A1- 2003 036 778

## Beschreibung

Die Erfindung betrifft eine Vorrichtung zur Defibrillation des Herzens mit einem implantierbaren kombinierten Herzschrittmacher und Defibrillator, mit zumindest einer Defibrillationselektrode und einer Gegenelektrode dafür sowie mit wenigstens einer ebenfalls implantierbaren Stimulations- und SensingElektrode, wobei die Defibrillationselektrode in Gebrauchsstellung von der Stimulationselektrode getrennt und subkutan nahe der Herzaußenseite im Bereich der Herzspitze implantierbar ist, wobei die Defibrillationselektrode wenigstens eine flexible Wendel aus Metall oder biokompatiblem Stahl ist.

Eine vergleichbare Vorrichtung ist aus der US 2003/036778 A1 bekannt. Die dabei vorgesehene Defibrillationselektrode kann unterschiedliche Formen und Abmessungen haben und beispielsweise eben oder mit einem kreisförmigen Querschnitt versehen sein. Dabei kann sie von einer Rolle oder Spule, als Geflecht oder Gewebe hergestellt sein. Für das Implantieren einer beispielsweise wendelförmigen Elektrode ist ein nach der Implantation wieder entfernbares Stillet erforderlich. Dabei muss dieses an der Elektrode angreifen, so dass nicht auszuschließen ist, dass eine wendelförmige Elektrode durch das Stillet aus seiner eigentlichen Gebrauchslage verformt und gedehnt werden kann.

Es besteht deshalb die Aufgabe, eine implantierbare Vorrichtung der eingangs genannten Art zu schaffen, bei welcher der Vorteil erhalten bleibt, dass der Thorax beim Implantieren der Defibrillationselektrode nicht geöffnet werden muss, und wobei eine Bruchgefahr oder eine Dehnung der Defibrillationselektrode vermieden werden kann.

Zur Lösung dieser Aufgabe ist die eingangs definierte Vorrichtung dadurch gekennzeichnet, dass an der als Wendel ausgebildeten Defibrillationselektrode für deren subkutane Implantation ein Zugelement oder Faden vorgesehen ist, dessen Angriff an der Defibrillationselektrode so gestaltet ist, dass die diese Defibrillationselektrode bildende Wendel beim Implantieren und bei einer auf das Zugelement oder den Faden wirkenden Zugkraft ungedehnt bleibt und dass das zum Implantieren der Defibrillationselektrode dienende Zugelement oder der Faden an einem die Wendel haltenden Träger angreift, welcher Träger die Zugkraft beim Implantieren aufnimmt und von der Wendel fern hält.

Dadurch ist es möglich, durch einen kleinen Schnitt in der Haut unterhalb der Rippen einen Zugang für diese relativ gering bemessene und flexible Defibrillationselektrode zu schaffen, die einerseits nahe der Herzaußenseite in dem dem Herzen benachbarten Gewebe eingelagert und andererseits mit ihrem Anschluss in bekannter Tunnelierungstechnik mit dem implantierten Herzschrittmacher und Defibrillator verbunden werden kann. Eine flexible Wendel lässt sich dabei bestmöglich an die anatomischen Verhältnisse anpassen und kann dennoch ausreichend große defibrillierende Stromstöße abgeben. Dabei bleibt der Vorteil erhalten, dass die Defibrillationselektrode nicht an der Außenseite des Herzens befestigt werden muss, also die normale Herzbewegung nicht beeinflusst wird.

Dadurch, dass an der als Wendel ausgebildeten Defibrillationselektrode für deren subkutane Implantation ein Zugelement oder Faden mit einem entsprechenden Angriff an der Defibrillationselektrode vorgesehen ist, bleibt die diese Defibrillationselektrode bildende Wendel beim Implantieren und bei einer auf das Zugelement oder den Faden wirkenden Zugkraft ungedehnt, wobei die Defibrillationselektrode anders als die Anordnung gemäß EP 0 602 356 A nicht am Herzen selbst, sondern in dem den Herzen benachbarten Gewebe angeordnet wird.

Somit kann mit Hilfe eines derartigen Fadens oder Zugelements, an welchem zunächst noch eine Nadel befestigt sein kann, die nach der Implantation abgetrennt wird, implantiert und in günstiger Position zur Herzaußenseite platziert werden, ohne dabei in ungewollter Weise verformt zu werden, so dass die die Defibrillationselektrode bildende Wendel trotz dieser Implantation mit Hilfe eines Zugelements und eines daran angreifenden Werkzeugs oder einer Nadel in ihrer vorgesehenen ungedehnten oder wenig gedehnten Form auch während der Implantation und danach verbleibt.

Das zum Implantieren der Defibrillationselektrode dienende Zugelement oder der Faden greift erfindungsgemäß an einem die Wendel haltenden Träger an, welcher Träger die Zugkraft beim Implantieren aufnimmt und von der von ihm getragenen oder gehaltenen Wendel fernhält, wodurch erreicht wird, dass die Defibrillationselektrode in Wendelform auch während des Implantierens nicht gedehnt werden muss. Dies stellt eine zweckmäßige Ausführungsform der Defibrillationselektrode in Wendelform dar, bei welcher die Wendel auch während des Implantierens nicht gedehnt werden muss.

Eine günstige Ausführungsform kann dabei vorsehen, dass das Zugelement oder der Faden am in Einführrichtung vorderen Endbereich oder Ende des Trägers befestigt ist. Somit wird die beim Implantieren ausgeübte Zugkraft auf den Träger übertragen, der seinerseits unter Zugkraft implantiert wird und dabei die an ihm befestigte Wendel sowie auch deren Zuleitung mitnimmt.

Eine abgewandelte Ausführungsform kann vorsehen, dass die als Defibrillationselektrode dienende Wendel in axialer Richtung in mehrere Wendelabschnitte unterteilt ist, die durch Leitungen miteinander verbunden sind. Die Wendel kann also mehrere Abschnitte haben, zwischen denen der oder die sie bildenden Drähte nicht gewendelt sind, was eine bessere Krümmung der Wendel insbesondere im Bereich der Herzspitze erlaubt, falls die anatomischen Verhältnisse dies erfordern.

Günstig ist es insbesondere auch für eine preiswerte Herstellung, wenn die Zuleitung zu der wendelförmigen Defibrillationselektrode eine isolierte niederohmige Litze oder Wendel ist.

Dadurch ist es in einfacher Weise möglich, dass die die Defibrillationselektrode bildende Wendel eine abisolierte Fortsetzung der wendelförmigen Zuleitung zu dieser Defibrillationselektrode ist. Es kann also einfach eine Wendel sowohl als Zuleitung als auch Defibrillationselektrode dadurch verwendet werden, dass das die Defibrillationselektrode bildende wendelförmige Ende abisoliert oder von vorneherein ohne Isolierung vorgesehen wird, während die Zuleitung dazu einfach dieselbe Wendel oder Mehrfachwendel mit Isolierung sein kann.

Innerhalb des die Zuleitung oder Wendel bildenden Drahtes kann eine die elektrische Leitfähigkeit erhöhende Silbermatrix oder Tantalmatrix vorgesehen sein. Somit kann die Leistung der Defibrillationselektrode auch bei relativ kleiner Abmessung entsprechend groß gestaltet werden.

Der die Defibrillationselektrode haltende Träger kann etwa die Länge der Elektrode haben oder eine etwas größere Länge als die Elektrode aufweisen. Insbesondere kann er sie in Einführrichtung etwas überragen, so dass das Anbringen eines Zugelements an diesem Träger ohne Beeinträchtigung der Wendel gut möglich ist.

Eine weitere Ausgestaltung der Erfindung kann vorsehen, dass die Defibrillationselektrode durch wenigstens zwei Wendeln gebildet ist, die über eine Leitungsverzweigung mit der Zuleitung verbunden sind. Auch dadurch kann der Vorteil erhalten bleiben, dass die Defibrillationselektrode hoch flexibel und dennoch mit geringen Abmessungen gestaltet ist und eine ideale Feldstärkenverteilung bei der Defibrillation erlaubt, so dass nur eine relativ niedrige Schockenergie notwendig ist, wobei gleichzeitig bei der Implantation nur ein minimaler subkutaner Eingriff erforderlich ist. Gleichzeitig bleibt der Vorteil sowohl bei nur einer als auch bei zwei Wendeln erhalten, dass diese Defibrillationselektrode von der Stimulationselektrode vollständig getrennt ist. Die hohe Flexibilität der Defibrillationselektrode und auch ihrer Zuleitung führt zu einer guten Bruchfestigkeit und entsprechend langen Lebensdauer.

Die wenigsten zwei die Defibrillationselektrode bildenden Wendeln können an einem gemeinsamen Träger insbesondere parallel zueinander verlaufend befestigt sein. Somit ist die Implantation mit Hilfe eines Zugelements und einer daran angreifenden Nadel, womit die Elektrode unterhalb des Herzens eingezogen werden kann, praktisch ebenso einfach wie die Implantation einer nur von einer Wendel gebildeten Defibrillationselektrode, wobei durch den Angriff des Zugelements oder Fadens an dem Träger eine ungewollte Dehnung der wendelförmigen Defibrillationselektrode auch vermieden wird, wenn diese zwei Wendeln aufweist.

Der zweckmäßigerweise flache oder etwa plattenförmig ausgebildete Träger kann in Gebrauchsstellung an der dem Herzen abgewandten Seite der Defibrillationselektrode angeordnet sein. Dadurch kann er gleichzeitig eine Abschirmung der eigentlichen Defibrillationselektrode auf deren dem Herz abgewandten Seite bilden. Entsprechend gezielt wird die Schockenergie der Defibrillationselektrode auf das Herz gerichtet.

Ihr Träger für die wendelförmige oder wendelförmigen Defibrillationselektroden kann aus isolierendem Werkstoff bestehen und als isolierende Abschirmung eine größere Breite als die Defibrillationselektrode oder -elektroden selbst haben und dieser gegenüber und gegenüber der oder den sie bildenden Wendeln seitlich - und wie bereits erwähnt auch in Längsrichtung - überstehen. Dadurch kann dieser auch als Abschirmung wirkende Träger die Lage der Defibrillations-elektrode innerhalb des subkutanen Gewebes in Gebrauchs-stellung stabilisieren.

Beispielsweise kann die Breite des als isolierende Abschirmung wirkenden Trägers doppelt oder dreimal oder viermal so groß wie die der Defibrillationselektrode sein, wobei aber auch ein Zwischenwert zwischen diesen Maßen möglich ist.

Ein günstiges Maßverhältnis für die die Defibrillationselektrode bildende Wendel oder Wendeln kann vorsehen, dass der Außendurchmesser dieser Wendel oder Wendeln wenigstens das Fünf-, Sechs- oder Siebenfache des Durchmessers des wendel- oder wendelnbildenden Drahtes oder einen Zwischenwert davon beträgt. Dies ergibt eine flexible Wendel mit günstiger. Außenabmessung, die eine ausreichend große Feldstärkeverteilung bei der Defibrillation und damit eine relativ niedrige Schockenergie ermöglicht.

Wichtig ist für eine erfolgreiche Defibrillation, wenn die Defibrillationselektrode und ihre Gegenelektrode(n) so platziert sind, dass der Defibrillationsstrom möglichst gleichmäßig durch das gesamte Herz fließt. Es ist also für eine möglichst gleichmäßige Feldstärkeverteilung während der elektrischen Defibrillation von Bedeutung, wie die Gegenelektrode der Defibrillationselektrode angeordnet ist.

Die Gegenelektrode für die Defibrillationselektrode kann dabei als transvenöse in das Herz einführbare Vorhofelektrode ausgebildet oder in Gebrauchsstellung außerhalb des Herzens implantierbar sein. Vor allem die zweite Alternative erlaubt eine bestmögliche Platzierung der Gegenelektrode relativ zur Lage der Defibrillationselektrode.

Günstig kann es sein, wenn die Gegenelektrode außerhalb des Herzens als Wendel im Herzschrittmacherbett und/oder an der Zuleitung der Defibrillationselektrode und/oder an der Zuleitung der Stimulationselektrode, insbesondere an deren Außenseite(n), angeordnet ist. Diese Zuleitungen verlaufen im Thorax in der Regel oberhalb oder im oberen Seitenbereich des Herzens, so dass sich mit einer Defibrillationselektrode im Bereich der Herzspitze eine gute Feldstärkeverteilung und ein das gesamte Herz durchströmender Defibrillationsstrom erreichen lässt.

Für eine möglichst einfache und preiswerte Lösung dieser Ausgestaltung der Erfindung kann es zweckmäßig sein, wenn die Zuleitung zu der Gegenelektrode innerhalb der Isolierung der Defibrillationselektrode oder der Stimulationselektrode angeordnet ist und bis zu der Gegenelektrode verläuft. Auf diese Weise kann die zweckmäßigerweise als Wendel an der Außenseite der Defibrillationselektrode oder der Stimulationselektrode angeordnete Gegenelektrode und ihre Zuleitung zu dem selben Stecker wie die entsprechende, sie tragende Elektrode führen und entsprechend einfach implantiert werden. Eine Vorhofelektrode braucht in diesem Falle also nicht implantiert zu werden, kann also eingespart werden.

Vor allem bei Kombination einzelner oder mehrerer der vorbeschriebenen Merkmale und Maßnahmen ergibt sich eine implantierbare Vorrichtung zur Defibrillation des Herzens, bei welcher die eigentliche Defibrillationselektrode aufgrund ihrer Wendelform platzsparend und hochflexibel mit großer Lebensdauer gestaltet werden kann, so dass sie durch einen minimalen Eingriff subkutan implantiert werden kann. Dabei kann sie an einer günstigen Stelle unterhalb des Herzens getrennt von der Stimulationselektrode angeordnet werden. Die Implantation ist sehr einfach mit Hilfe einer Nadel und eines Zugelements möglich, wobei die dabei auftretenden Zugkräfte aber von der Wendel selbst ferngehalten bleiben.

Nachstehend sind Ausführungsbeispiele der Erfindung anhand der Zeichnung näher beschrieben. Es zeigt in zum Teil schematisierter Darstellung:

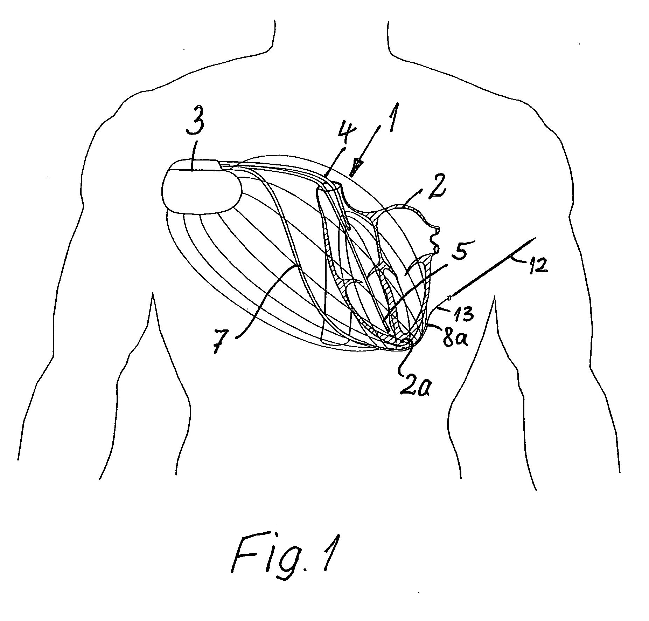

- Fig. 1: eine implantierte erfindungsgemäße Vorrichtung zur Defibrillation mit einem in Gebrauchsstellung angeordneten kombinierten Herzschrittmacher und Defibrillator, einer davon ausgehenden, transvenös in das Herz eingeführten Vorhofelektrode, einer ebenfalls in das Herz führenden Stimulations- und Sensing-Elektrode und einer subkutan nahe der Herzaußenseite im Bereich der Herzspitze implantierten Defibrillationselektrode kurz nach der Implantation und noch vor dem Abtrennen der zum Implantieren dienenden, an einem Zugelement angreifenden Nadel, wobei die Feldstärkeverteilung am Herzen schematisiert angedeutet ist,
- Fig. 2: in vergrößertem Maßstab einen Längsschnitt durch das Herz und dabei die Anordnung der Vorhofelektrode, der Stimulationselektrode und der nahe der Außenseite im Bereich der Herzspitze implantierten Defibrillationselektrode, wobei die zum subkutanen Implantieren dienende Nadel noch nicht von dem Faden oder Zugelement abgetrennt ist,
- Fig. 3: eine Ansicht einer erfindungsgemäßen, als Wendel aus Metall oder biokompatiblem Stahl ausgebildeten Defibrillationselektrode mit Zuleitung und Zugelement, an welchem eine gekrümmte Nadel angreift,
- Fig. 4: eine der Fig. 3 entsprechende Darstellung jedoch mit einer geradlinigen Nadel,
- Fig. 5: eine Teil-Darstellung der erfindungsgemäßen Defibrillationselektrode, bei welcher parallele Wendeln an einem gemeinsamen Träger angeordnet sind, an welchem das Zugelement oder der Faden für die Nadel angreifen,
- Fig. 6: eine Seitenansicht der wendelförmigen Defibrillationselektrode und des sie tragenden Trägers mit dem schematisierten Angriff des Zugelements oder Fadens an dem in Implantationsrichtung vorderen Ende und der Verbindung zu der Zuleitung an dem entgegengesetzten Ende,
- Fig. 7: eine der Fig. 1 entsprechende Darstellung, wobei jedoch an Stelle einer Vorhofelektrode als Gegenelektrode zu der Defibrillationselektrode eine Wendel vorgesehen ist, die über eine Zuleitung mit dem Herzschrittmacher und Defibrillator verbunden ist und sich außerhalb des Herzens befindet,
- Fig. 8: eine der Fig. 7 entsprechende Darstellung und Anordnung, bei welcher jedoch die wendelförmige Gegenelektrode für die Stimulationselektrode auf deren Zuleitung außenseitig angeordnet ist, sowie
- Fig. 9: in vergrößertem Maßstab die auf der Außenseite der Zuleitung der Defibrillationselektrode befindliche wendelförmige Gegenelektrode.

Eine in Fig. 1 im Ganzen mit 1 bezeichnete Vorrichtung dient zur Defibrillation eines Herzens 2 und umfasst einen implantierbaren kombinierten Herzschrittmacher und Defibrillator 3, eine transvenös in das Herz einführbare Vorhofelektrode 4, eine implantierbare Stimulations- und Sensing-Elektrode 5 und eine im Ganzen mit 6 bezeichnete Defibrillationselektrode, die wie die übrigen Elektroden über eine Zuleitung 7 mit dem Defibrillator 3 in Gebrauchsstellung verbunden ist und subkutan nahe der Außenseite im Bereich der Herzspitze 2a implantiert ist. Dabei erkennt man in Fig. 1 die Feldstärkeverteilung beziehungsweise den Stromfluss im Falle eines Defibrillationsvorganges, der das Herz möglichst allseitig umschließt und umfasst.

Dabei erkennt man anhand der schematisiert angedeuteten Feldlinien, dass die Vorhofelektrode 4 eine Gegenelektrode zu der Defibrillationselektrode 6 bildet.

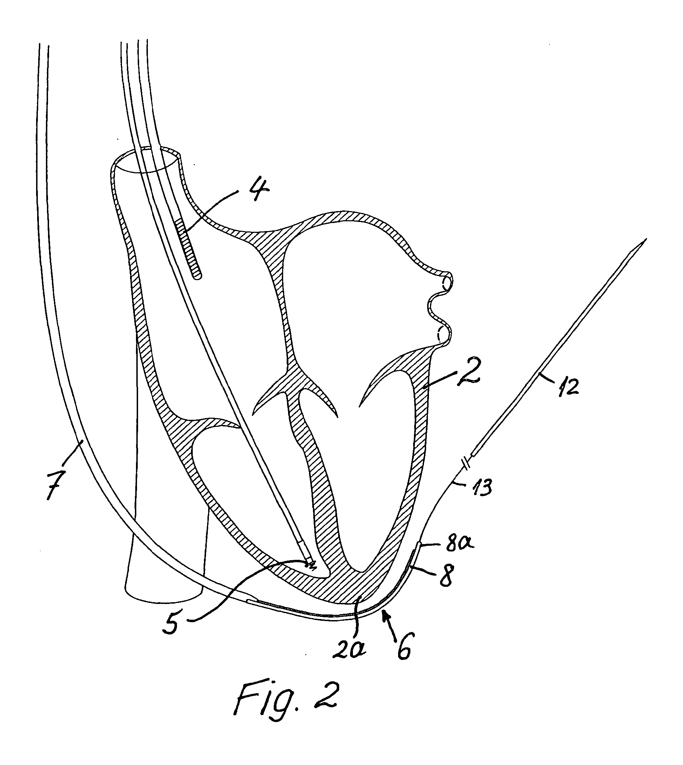

Vor allem in Fig. 2 wird deutlich, dass dabei die Defibrillationselektrode 6 zum Umfassen der Herzaußenseite im Bereich der Herzspitze 2a zweckmäßigerweise flexibel ist, um die Krümmung der Herzspitze wenigstens teilweise berücksichtigen zu können.

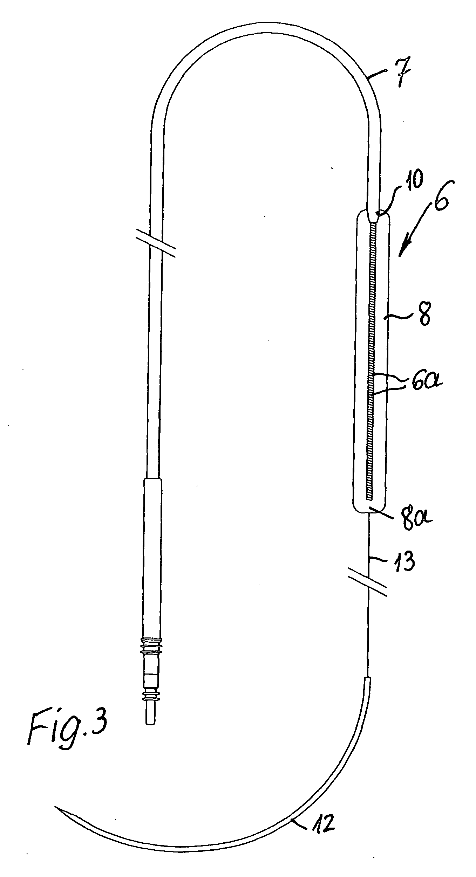

Dazu ist die Defibrillationselektrode 6 gemäß den Figuren 3 bis 6 eine flexible Wendel aus Metall oder biokompatiblem Stahl mit wenigstens einem schraubenförmig gewundenen Draht 6a oder auch zwei oder mehr parallelen gewundenen Drähten 6a, was die gewünschte Biegsamkeit und Flexibilität begünstigt.

Aus allen Figuren geht hervor, dass an der als Wendel ausgebildeten Defibrillationselektrode 6 für deren subkutane Implantation ein Zugmittel oder Faden 13 in noch zu beschreibender Weise angreift, dessen Angriff an der Defibrillationselektrode 6 so gestaltet ist, dass die diese bildende Wendel beim Implantieren und bei einer auf das Zugelement oder den Faden 13 wirkenden Zugkraft ungedehnt bleibt, also ihre in den Figuren 3 bis 6 erkennbare Wendelform weitestgehend unverändert beibehält. Lediglich die Krümmung gemäß Fig. 1 und 2 kann und soll die Wendel bei der Implantation erhalten.

Um die Wendel und somit die Defibrillationselektrode 6 von dem von dem Zugelement oder Faden 13 ausgehenden Kräften freizuhalten, greift dieses Zugelement beziehungsweise der Faden 13 gemäß allen Figuren in den Ausführungsbeispielen an einem die Wendel haltenden Träger 8 an, welcher Träger 8 die Zugkraft beim Implantieren aufnimmt und somit von der Wendel 6 fernhält. Dabei erkennt man, dass das Zugelement oder der Faden 13 am in Einführrichtung vorderen Endbereich oder Ende 8a des Trägers 8 befestigt ist.

In allen Ausführungsbeispielen ist als Defibrillations-elektrode 6 eine praktisch durchgehende Wendel vorgesehen. Diese könnte aber in axialer Richtung auch aus mehreren Wendelabschnitten gebildet sein, welche Abschnitte dann durch Leitungsstücke miteinander verbunden sind, um unter Umständen eine noch höhere Flexibilität zu ermöglichen.

Die in den Figuren 1 bis 4 gut erkennbare Zuleitung 7 zu der wendelförmigen Defibrillationselektrode 6 kann dabei eine isolierte niederohmige Litze oder ebenfalls eine Wendel sein. Die Isolierung 10 ist dabei in den Figuren 3 bis 6 dadurch gut zu erkennen, dass die die Defibrillationselektrode bildende Wendel eine abisolierte Fortsetzung der innerhalb der Isolierung 10 befindlichen wendelförmigen Zuleitung 7 zu der Defibrillationselektrode 6 ist, was die Herstellung der gesamten Anordnung begünstigt. Dabei kann innerhalb des die Zuleitung 7 und/oder die Wendel 6 bildenden Drahtes eine die elektrische Leitfähigkeit erhöhende Silbermatrix oder Tantalmatrix vorgesehen sein.

Vor allem in den Figuren 3 bis 6 erkennt man, dass der die Defibrillationselektrode 6 haltende Träger 8 die Länge der Elektrode 6 übertrifft, so dass diese entsprechend sicher gehalten wird.

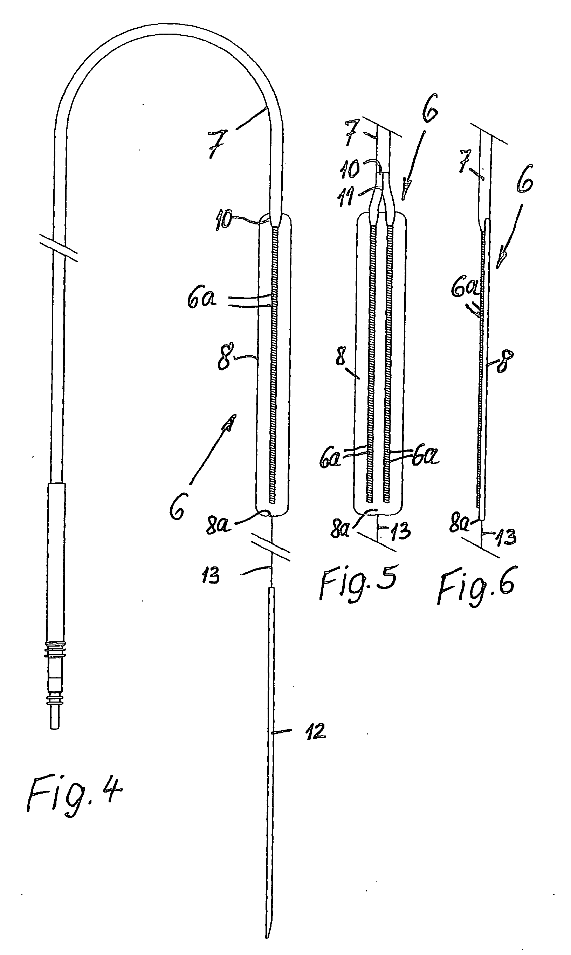

In Fig. 5 ist dargestellt, dass die Defibrillationselektrode 6 auch durch wenigstens zwei Wendeln gebildet sein kann, die über eine Leitungsverzweigung 11 mit der Zuleitung 9 verbunden sind und dadurch auf engem Raum und platzsparend dennoch die Wirksamkeit der Defibrillationselektrode 6 erhöhen können.

Diese beiden die Defibrillationselektrode 6 bildenden Wendeln sind dabei an dem gemeinsamen Träger 8 parallel zueinander verlaufend befestigt, so dass das Zugelement oder der Faden 13 an dem gegenüber den Wendeln überstehenden Ende 8a dieses Trägers 8 in der schon beschriebenen Weise angreifen kann und Zugkräfte an diesem Zugelement 13 die Wendeln nicht verformen.

In Fig. 6 erkennt man dabei, dass die Wendel oder Wendeln von diesem Träger 8 an einer Seite gehalten und nach dieser Seite auch abgeschirmt werden, so dass dieser flache oder etwa plattenförmig ausgebildete Träger 8 in Gebrauchsstellung gemäß Fig. 1 und 2 an der dem Herzen 2 abgewandten Seite der Defibrillationselektrode 6 angeordnet und zur Abschirmung benutzt werden kann. Entsprechend gut wirkt sich die Defibrillationselektrode 6 auf das Herz 2 aus.

Der Träger 8 für diese wendelförmige Defibrillationselektrode 6 besteht dabei zweckmäßigerweise aus isolierendem Werkstoff und hat als isolierende Abschirmung gemäß den Figuren 3 bis 5 eine größere Breite als die Defibrillationselektrode 6 selbst, auch wenn diese aus 2 Wendeln gebildet ist, und steht gegenüber der Defibrillationselektrode 6 und den sie bildenden Wendeln auch seitlich über, um eine entsprechend wirkungsvolle Abschirmung zu bilden. Aufgrund der flachen oder plattenförmigen Ausbildung und der Wahl eines entsprechend biegsamen Werkstoffs kann auch der Träger 8 gut gekrümmt und an die anatomischen Verhältnisse angepasst werden, ist also ebenfalls wie die Wendel oder Wendeln entsprechend flexibel.

Die Breite des als isolierende Abschirmung wirkenden Trägers 8 ist dabei zum Beispiel doppelt oder dreimal oder viermal so groß wie die der Defibrillationselektrode 6 selbst. Der Außendurchmesser der die Defibrillationselektrode 6 bildenden Wendel oder Wendeln kann wenigstens das Fünf-, Sechs- oder Siebenfache des Durchmessers des die Wendel oder Wendeln bildenden Drahtes oder auch einen Zwischenwert davon betragen.

Insgesamt ergibt sich eine implantierbare Vorrichtung 1 zur Defibrillation des Herzens, deren Defibrillationselektrode 6 eine verbesserte Zuverlässigkeit und insbesondere eine höhere Bruchsicherheit hat. Aufgrund der hoch flexiblen Gestaltung mit relativ geringen Abmessungen, wobei der Durchmesser der Wendel oder Wendeln etwa dreiviertel bis ein Millimeter, insbesondere 0,8 bis 0,9 Millimeter betragen kann, ist eine Implantation der Defibrillationselektrode 6 mit ihrem Träger 8 durch einen minimalen subkutanen Eingriff möglich, bei welcher der Faden 13 mit Hilfe einer Nadel 12 durch das dem Herzen 2 nahe Gewebe gezogen werden kann, wonach die Nadel 12, die gemäß Fig. 3 gekrümmt oder gemäß Fig. 4 geradlinig sein kann, einfach abgetrennt wird. Die Defibrillationselektrode ist dabei von der Stimulationselektrode 5 vollständig getrennt und die Wendelform auch der Zuleitung 7 ergibt eine hochflexible und bruchfeste Zuleitung 7 mit langer Lebensdauer. Die Verbindung zum Defibrillator 3 mit Hilfe der Zuleitung 7 erfolgt nach dem Einziehen mit Hilfe der Nadel 12 unterhalb des Herzens 2 durch die bekannte Tunnelierungs-Methode.

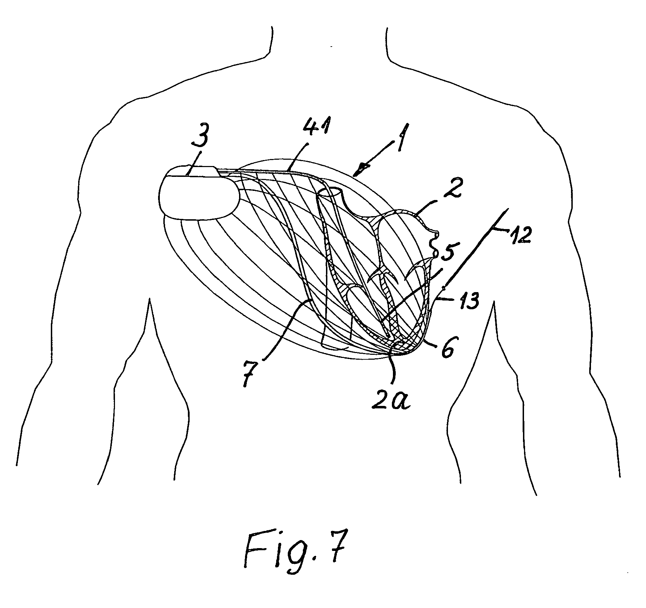

In den Fig. 7 bis 9 ist eine bezüglich der Gegenelektrode zu der Defibrillationselektrode 6 abgewandelte Anordnung dargestellt, wobei diese Gegenelektrode jeweils außerhalb des Herzens 2 implantierbar oder implantiert ist.

In Fig. 7 erkennt man eine Gegenelektrode 41, die an der Zuleitung der Stimulationselektrode 5 außerhalb des Herzens an der Außenseite dieser Zuleitung angeordnet ist und mit ihrer eigenen Zuleitung zu einem Stecker in den Herzschrittmacher und Defibrillator 3 führt. Auch dadurch lässt sich eine gute Feldstärkeverteilung und Stromleitung durch das Herz 2 erzielen, wie schematisiert durch entsprechende Feldlinien angedeutet ist.

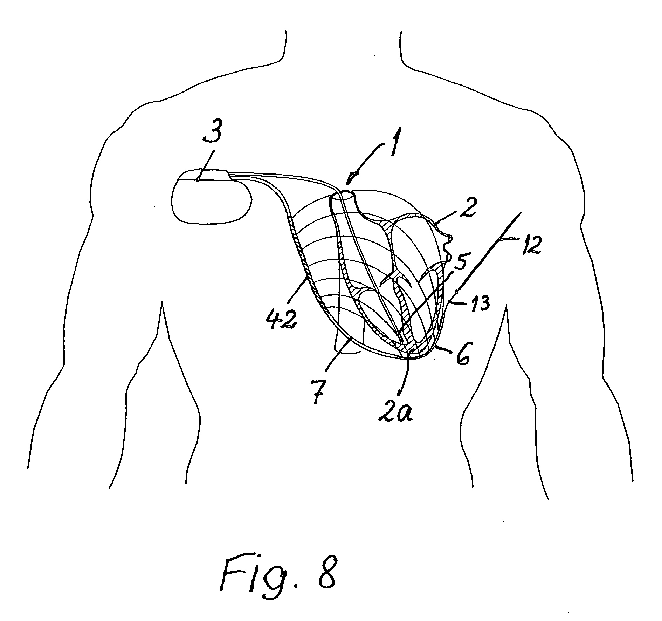

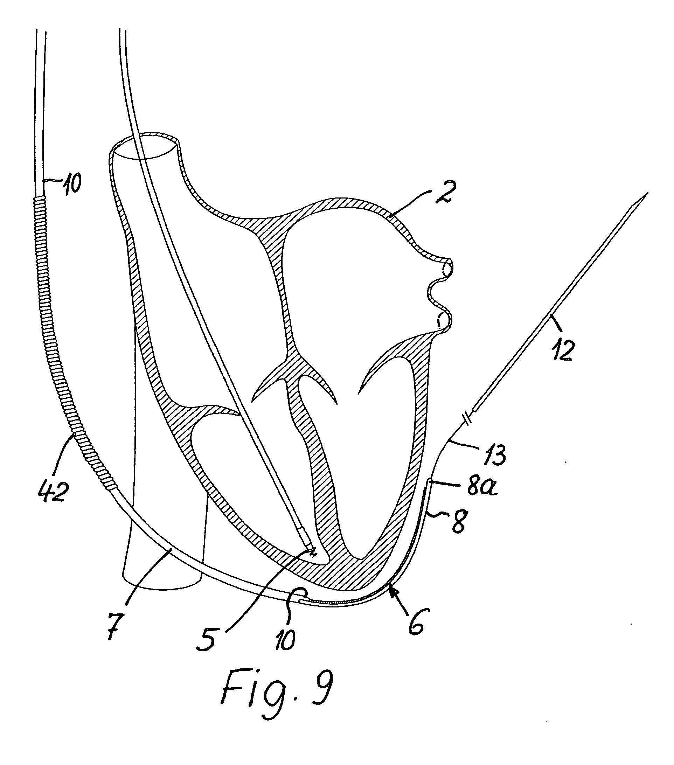

Fig. 8 zeigt demgegenüber eine abgewandelte Ausführungsform, bei welcher die Gegenelektrode 42 ebenfalls außerhalb des Herzens 2 angeordnet ist und dabei an der Zuleitung 7 der Defibrillationselektrode 6 an deren Außenseite angeordnet ist, was gemäß Fig. 8 und den dargestellten Feldlinien zu einer optimalen Feldstärkeverteilung und einem optimalen Stromfluss durch das Herz 2 führt. Dabei ist in Fig. 9 diese Anordnung noch vergrößert dargestellt, so dass man deutlich die wendelförmige Gegenelektrode 42 auf der Außenseite der Isolierung 10 der Zuleitung 7 erkennt, wobei die Zuleitung zu dieser Gegenelektrode 42 ebenso wie die zu der Gegenelektrode 41 in Fig. 7 in der Zeichnung nicht erkennbar ist, weil sie innerhalb der Isolierung der entsprechenden Zuleitung, gemäß Fig. 9 innerhalb der Isolierung 10 der Zuleitung 7 der Defibrillationselektrode 6, angeordnet ist und von der Gegenelektrode 42 oder 41 zu dem Herzschrittmacher und Defibrillator 3 verläuft.

Die Vorrichtung 1 dient zur Defibrillation des Herzens 2 und ist insgesamt implantierbar. Sie weist einen implantierbaren kombinierten Herzschrittmacher und Defibrillator 3, wenigstens eine Defibrillationselektrode 6 und eine Gegenelektrode 4, 41 oder 42 dafür sowie eine ebenfalls implantierbare Stimulations- und Sensing-Elektrode 5 auf, wobei die Defibrillationselektrode 6 subkutan nahe der Herzaußenseite im Bereich der Herzspitze 2a beispielsweise mit Hilfe eines Zugelements 13 und einer Nadel 12 einziehbar und implantierbare ist und als wenigstens eine flexible Wendel aus Metall oder biokompatiblem Stahl ausgestaltet ist, also eine hohe Flexibilität und einen geringen Platzbedarf hat.

## Patentansprüche

1. Vorrichtung (1) zur Defibrillation des Herzens (2) mit einem implantierbaren kombinierten Herzschrittmacher und Defibrillator (3), mit zumindest einer Defibrillationselektrode (6) und einer Gegenelektrode (4, 41, 42) dafür sowie mit wenigstens einer ebenfalls implantierbaren Stimulations- und Sensing-Elektrode (5), wobei die Defibrillationselektrode (6) in Gebrauchsstellung von der Stimulationselektrode (5) getrennt und subkutan nahe der Herzaußenseite im Bereich der Herzspitze (2a) implantierbar ist, wobei die Defibrillationselektrode (6) wenigstens eine flexible Wendel aus Metall oder biokompatiblem Stahl mit wenigstens einem schraubenförmig gewundenen Draht (6a) ist und an der als Wendel ausgebildeten Defibrillations-elektrode (6) für deren subkutane Implantation ein Faden (13) vorgesehen ist, dessen Angriff an der Defibrillationselektrode (6) so gestaltet ist, dass die diese Defibrillationselektrode bildende Wendel beim Implantieren und bei einer auf den Faden (13) wirkenden Zugkraft ungedehnt bleibt, **dadurch gekennzeichnet, dass** der Faden (13) an einem die Wendel haltenden Träger (8) angreift, welcher Träger (8) die Zugkraft beim Implantieren aufnimmt und von der Wendel fern hält.

2. Vorrichtung nach Anspruch 1, **dadurch gekennzeichnet, dass** der Faden (13) am in Einführrichtung vorderen Endbereich oder Ende (8a) des Trägers (8) angreift oder befestigt ist.

3. Vorrichtung nach einem der Ansprüche 1 oder 2, **dadurch gekennzeichnet, dass** die als Defibrillationselektrode (6) dienende Wendel in axialer Richtung in mehrere Wendelabschnitte unterteilt ist, die durch Leitungen miteinander verbunden sind.

4. Vorrichtung nach einem der Ansprüche 1 bis 3, **dadurch gekennzeichnet, dass** die Zuleitung (7) zu der wendelförmigen Defibrillationselektrode (6) eine isolierte niederohmige Litze oder Wendel ist.

5. Vorrichtung nach einem der Ansprüche 1 bis 4, **dadurch gekennzeichnet, dass** die die Defibrillationselektrode (6) bildende Wendel eine abisolierte Fortsetzung der wendelförmig ausgebildeten Zuleitung (7) zu dieser Defibrillationselektrode (6) ist.

6. Vorrichtung nach einem der Ansprüche 1 bis 5, **dadurch gekennzeichnet, dass** innerhalb des die Zuleitung (7) oder Wendel (6) bildenden Drahtes eine die elektrische Leitfähigkeit erhöhende Silbermatrix oder Tantalmatrix vorgesehen ist.

7. Vorrichtung nach einem der Ansprüche 1 bis 6, **dadurch gekennzeichnet, dass** der die Defibrillationselektrode (6) haltende Träger (8) etwa die Länge der Elektrode (6) hat oder eine etwas größere Länge als die Elektrode (6) aufweist.

8. Vorrichtung nach einem der Ansprüche 1 bis 7, **dadurch gekennzeichnet, dass** die Defibrillationselektrode (6) durch wenigstens zwei Wendeln gebildet ist, die über eine Leitungsverzweigung (11) mit der Zuleitung (7) verbunden sind.

9. Vorrichtung nach einem der Ansprüche 1 bis 8, **dadurch gekennzeichnet, dass** die wenigstens zwei die Defibrillationselektrode (6) bildenden Wendeln an einem gemeinsamen Träger (8) insbesondere parallel zueinander verlaufend angeordnet oder befestigt sind.

10. Vorrichtung nach einem der Ansprüche 1 bis 9, **dadurch gekennzeichnet, dass** der Träger (8) flach oder etwa plattenförmig und flexibel ausgebildet und an einer Seite der Defibrillationselektrode (6) angeordnet ist, die in Gebrauchsstellung dem Herzen (2) abgewandt ist.

11. Vorrichtung nach einem der Ansprüche 1 bis 10, **dadurch gekennzeichnet, dass** der Träger (8) für die wendelförmige oder wendelförmigen Defibrillationselektrode (6) aus isolierendem Werkstoff besteht und als isolierende Abschirmung eine größere Breite als die Defibrillationselektrode (6) selbst hat und dieser gegenüber und gegenüber der oder den sie bildenden Wendeln seitlich übersteht.

12. Vorrichtung nach einem der Ansprüche 1 bis 11, **dadurch gekennzeichnet, dass** die Breite des als isolierende Abschirmung wirkenden Trägers (8) doppelt oder dreimal oder viermal so groß wie die der Defibrillationselektrode (6) ist.

13. Vorrichtung nach einem der Ansprüche 1 bis 12, **dadurch gekennzeichnet, dass** der Außendurchmesser der die Defibrillationselektrode (6) bildenden Wendel oder Wendeln wenigstens das Fünf-, Sechs- oder Siebenfache des Durchmessers des die Wendel oder Wendeln bildenden Drahtes oder einen Zwischenwert davon, beispielsweise dreiviertel bis ein Millimeter, insbesondere 0,8 bis 0,9 Millimeter, beträgt.

14. Vorrichtung nach einem der Ansprüche 1 bis 13, **dadurch gekennzeichnet, dass** die Gegenelektrode (4, 41, 42) für die Defibrillationselektrode (6) als transvenös in das Herz (2) einführbare Vorhofelektrode ausgebildet oder in Gebrauchsstellung außerhalb des Herzens (2) implantierbar ist.

15. Vorrichtung nach einem der Ansprüche 1 bis 14, **dadurch gekennzeichnet, dass** die Zuleitung zu der Gegenelektrode (41, 42) innerhalb der Isolierung der Zuleitung (7) der Defibrillationselektrode (6) oder der Zuleitung der Stimulationselektrode (5) angeordnet ist und von der Gegenelektrode zu dem Herzschrittmacher und Defibrillator (3) verläuft.

## Claims

1. Device (1) for the defibrillation of the heart (2) comprising an implantable, combination cardiac pacemaker and defibrillator (3), with at least one defibrillation electrode (6) and a counter electrode (4, 41, 42) for the defibrillation electrode, and also with at least one implantable stimulation and sensing electrode (5), wherein, in a position of use, the defibrillation electrode (6) is separated from the stimulation electrode (5) and is adapted to be implanted subcutaneously close to an outside of the heart in a region of the cardiac apex (2a), and the defibrillation electrode (6) is at least one flexible helix made from metal or biocompatible steel with at least one helically wound wire (6a) and a tensioning element or thread (13) is provided on the defibrillation electrode (6) for its subcutaneous implantation, wherein an attachment of the tension element or the thread to the defibrillation electrode (6) is formed so that the at least one flexible helix forming the defibrillation electrode remains undilated during implantation and when there is a tensile force acting on the tension element or the thread (13), **characterized in that** the tension element or thread (13) attaches to a carrier (8) holding the helix, wherein the carrier (8) absorbs the tensile force during the implantation procedure and the force is kept away from the helix.

2. Device according to claim 1, wherein the tension element or the thread (13) attaches to or is mounted on a front end region or end (8a) of the carrier (8) in the insertion direction.

3. Device according to claim 1 or 2, **characterized in that** the at least one flexible helix used as the defibrillation electrode (6) is divided in an axial direction into several helix sections that are connected to each other by conductors.

4. Device according to any one of claims 1 to 3, **characterized in that** the feed line (7) to the defibrillation electrode (6) is an insulated, low-impedance braid or helix.

5. Device according to any one of claims 1 to 4, **characterized in that** the at least one flexible helix forming the defibrillation electrode (6) is an insulation-stripped projection of the feed line (7) to the defibrillation electrode (6).

6. Device according to any one of claims 1 to 5, **characterized in that** within the wire forming the feed line (7) or helix (6), there is a silver matrix or tantalum matrix increasing electrical conductivity.

7. Device according to any one of claims 1 to 6, **characterized in that** the carrier (8) holding the defibrillation electrode (6) has approximately the same length as the electrode (6) or a somewhat greater length than the electrode (6).

8. Device according to any one of claims 1 to 7, **characterized in that** the defibrillation electrode (6) is formed by at least two helices that are connected to the feed line (7) by a wire branching point (11).

9. Device according to any one of claims 1 to 8, **characterized in that** the two or more helices forming the defibrillation electrode (6) are arranged or mounted parallel to each other on the common carrier (8).

10. Device according to any one of claims I to 9, **characterized in that** the carrier (8) is flat or somewhat plate-shaped and flexible, and is arranged, in a position of use, on a side of the defibrillation electrode (6) facing away from the heart (2).

11. Device according to any one of claims 1 to 10, **characterized in that** the carrier (8) for the defibrillation electrode (6) is made from insulating material and has, as insulating shielding, a greater width than the defibrillation electrode (6) itself and projects laterally past the helix or helices forming them.

12. Device according to any one of claims 1 to 11, **characterized in that** the width of the carrier (8) acting as the insulating shielding is two-times or three-times or four-times as large as a width of the defibrillation electrode (6).

13. Device according to any one of claims 1 to 12, **characterized in that** an outer diameter of the flexible helix or helices forming the defibrillation electrode (6) equals at least five-times, six-times, or seven-times the diameter of the wire forming the flexible helix or helices, or equals an intermediate value thereof, for example three-fourths to one millimetre, in particular 0.8 to 0.9 millimetre.

14. Device according to any one of claims 1 to 13, **characterized in that** the counter electrode (4, 41, 42) for the defibrillation electrode (6) is formed as an atrial electrode that is adapted to be inserted transvenously into the heart (2) or can be implanted into the position of use outside of the heart (2).

15. Device according to any one of claims 1 to 14, **characterized in that** a feed line to the counter electrode (41, 42) is arranged within insulation of the feed line (7) of the defibrillation electrode (6) or the feed line of the stimulation electrode (5) and runs from the counter electrode to the cardiac pacemaker and defibrillator (3).

## Revendications

1. Dispositif (1) dévolu à la défibrillation du coeur (2), comprenant un stimulateur cardiaque et défibrillateur combiné (3) implantable, au moins une électrode de défibrillation (6) et une contre-électrode (4, 41, 42) associée à cette dernière, ainsi qu'au moins une électrode (5) de stimulation et de détection, semblablement implantable, sachant qu'en position d'utilisation, ladite électrode de défibrillation (6) est séparée d'avec ladite électrode de stimulation (5) et peut être implantée par voie sous-cutanée à proximité de la face extérieure du coeur, dans la région de l'apex cardiaque (2a), ladite électrode de défibrillation (6) se présentant au moins comme un enroulement flexible en métal ou en acier biocompatible, comportant au moins un fil métallique (6a) torsadé hélicoïdalement, et sachant qu'il est prévu sur ladite électrode de défibrillation (6) réalisée sous la forme d'un enroulement, en vue de l'implantation sous-cutanée de celle-ci, un fil (13) dont le rattachement à ladite électrode de défibrillation (6) est conçu de façon telle que l'enroulement, constituant cette électrode de défibrillation, demeure exempt d'allongement au cours de l'implantation et en présence d'une force de traction agissant sur ledit fil (13), **caractérisé par le fait que** le fil (13) est en prise avec un support (8) retenant l'enroulement, lequel support (8) absorbe la force de traction lors de l'implantation, et la maintient éloignée dudit enroulement.

2. Dispositif selon la revendication 1, **caractérisé par le fait que** le fil (13) est rattaché ou fixé à la région extrême ou à l'extrémité (8a) du support (8) qui est située à l'avant dans la direction d'insertion.

3. Dispositif selon l'une des revendications 1 ou 2, **caractérisé par le fait que** l'enroulement remplissant la fonction d'électrode de défibrillation (6) est scindé, dans le sens axial, en plusieurs segments raccordés les uns aux autres par des conducteurs.

4. Dispositif selon l'une des revendications 1 à 3, **caractérisé par le fait que** le conducteur d'alimentation (7), gagnant l'électrode de défibrillation (6) en forme d'enroulement, est un cordon ou un enroulement isolé à faible valeur ohmique.

5. Dispositif selon l'une des revendications 1 à 4, **caractérisé par le fait que** l'enroulement, constituant l'électrode de défibrillation (6), est un prolongement isolé du conducteur d'alimentation (7) réalisé en forme d'enroulement et gagnant cette électrode de défibrillation (6).

6. Dispositif selon l'une des revendications 1 à 5, **caractérisé par le fait qu'**une matrice d'argent ou une matrice de tantale, accroissant la conductivité électrique, est prévue à l'intérieur du fil métallique constituant le conducteur d'alimentation (7) ou l'enroulement (6).

7. Dispositif selon l'une des revendications 1 à 6, **caractérisé par le fait que** le support (8), retenant l'électrode de défibrillation (6), présente à peu près la longueur de ladite électrode (6) ou une longueur sensiblement supérieure à celle de ladite électrode (6).

8. Dispositif selon l'une des revendications 1 à 7, **caractérisé par le fait que** l'électrode de défibrillation (6) est composée d'au moins deux enroulements raccordés au conducteur d'alimentation (7) par l'intermédiaire d'une bifurcation conductrice (11).

9. Dispositif selon l'une des revendications 1 à 8, **caractérisé par le fait que** les enroulements au nombre minimal de deux, qui constituent l'électrode de défibrillation (6), sont disposés ou fixés sur un support commun (8) et s'étendent, en particulier, avec parallélisme mutuel.

10. Dispositif selon l'une des revendications 1 à 9, **caractérisé par le fait que** le support (8) est de réalisation flexible et aplatie, ou sensiblement en forme de platine, et est situé sur un côté de l'électrode de défibrillation (6) qui est tourné à l'opposé du coeur (2) en position d'utilisation.

11. Dispositif selon l'une des revendications 1 à 10, **caractérisé par le fait que** le support (8), dédié à l'électrode ou aux électrodes de défibrillation (6) en forme d'enroulement(s), consiste en un matériau isolant ; présente, en tant que blindage isolant, une largeur supérieure à celle de l'électrode de défibrillation (6) proprement dite ; et fait saillie latéralement par rapport à cette dernière et par rapport à l'enroulement ou aux enroulements qui la constitue(nt).

12. Dispositif selon l'une des revendications 1 à 11, **caractérisé par le fait que** la largeur du support (8), agissant comme un blindage isolant, représente le double ou le triple, voire le quadruple de celle de l'électrode de défibrillation (6).

13. Dispositif selon l'une des revendications 1 à 12, **caractérisé par le fait que** le diamètre extérieur de l'enroulement ou des enroulements constituant l'électrode de défibrillation (6) représente au moins le quintuple, le sextuple ou le septuple du diamètre du fil métallique matérialisant ledit ou lesdits enroulement(s), ou présente une valeur intermédiaire, par exemple de trois quarts de millimètre à un millimètre, notamment de 0,8 à 0,9 millimètre.

14. Dispositif selon l'une des revendications 1 à 13, **caractérisé par le fait que** la contre-électrode (4, 41, 42), associée à l'électrode de défibrillation (6), est réalisée sous la forme d'une électrode auriculaire pouvant être insérée dans le coeur (2) par voie transveineuse, ou peut être implantée à l'extérieur du coeur (2) en position d'utilisation.

15. Dispositif selon l'une des revendications 1 à 14, **caractérisé par le fait que** le conducteur d'alimentation gagnant la contre-électrode (41, 42) est logé à l'intérieur de l'isolation du conducteur d'alimentation (7) de l'électrode de défibrillation (6), ou du conducteur d'alimentation de l'électrode de stimulation (5), et s'étend depuis ladite contre-électrode jusqu'au stimulateur cardiaque et défibrillateur (3).
